# EUROPEAN PATENT SPECIFICATION

(11) **EP 0 691 958 B1**
(45) Date of publication and mention of the grant of the patent: **13.08.1997**
(21) Application number: 94912016.6
(22) Date of filing: 05.04.1994
(51) Int. Cl.: C07D 213/38, A61K 31/44

(54) **(S)-ALPHA-PHENYL-2-PYRIDINEETHANAMINE BENZOATE AND ITS USE AS A MEDICAMENT**
(S)-ALPHA-PHENYL-2-PYRIDINETHANAMIN BENZOAT UND SEINE VERWENDUNG ALS ARZNEIMITTEL
BENZOATE (S)-ALPHA-PHENYL-2-PYRIDINEETHANAMINE ET SON UTILISATION EN TANT QUE MEDICAMENT

(30) Priority: 01.04.1993 WO PCT/GB93/00689
(43) Date of publication of application: 17.01.1996
(73) Proprietor: Astra Aktiebolag, 151 85 Södertälje (SE)
(72) Inventor: MURRAY, Robert, John, Brighton, NY 14618 (US); MATHISEN, Donald, Fairport, NY 14450 (US)
(86) International application number: GB9400716
(87) International publication number: WO9422832

(56) References cited:
- EP-A- 0 356 035
- EP-A- 0 472 325
- WO-A-93/20052
- US-A- 3 422 191
- J.B. TAYLOR 'Comprehensive medicinal chemistry Vol.5 Biopharmaceutics' , PERGAMON PRESS , OXFORD * page 379 lines 9-13 *

## Description

This invention relates to (*S*)-α-phenyl-2-pyridineethanamine benzoate, its use as a pharmaceutical, in particular in the treatment of neurodegenerative disorders, a process for its production, and pharmaceutical formulations including it.

A major problem with existing drugs used to treat neurodegenerative disorders is a lack of predictability in the concentration of a drug in a patient's blood plasma resulting from administration of a given quantity of that drug, *i.e*. existing drugs do not exhibit linear pharmacokinetics. It has been stated that an ideal drug in this field would show a linear relationship between blood plasma concentration and dose size so that a given change in dose would yield a predictable change in blood plasma concentration of the drug ['Pharmacokinetics of old, new and yet-to-be discovered antiepileptic drugs', R H Levy and B M Kerr, Epilepsia, vol 30, Supp 1, S35-S41, 1989].

European Patent Application 356035 discloses a large number of compounds for use in the treatment of neurodegenerative disorders, including α-phenyl-2-pyridineethanamine [referred to therein as 1-phenyl-2-(2-pyridinyl)ethylamine]. The (*S*)-enantiomer of this compound, and its pharmaceutically acceptable acid addition salts, have been found to exhibit linear pharmacokinetics, and are disclosed in WO 93/20052 (International Patent Application N° PCT/GB93/00689).

Surprisingly, it has now been found that the benzoate salt of (*S*)-α-phenyl-2-pyridine-ethanamine possesses a number of advantages, including outstanding stability to moisture.

Thus, according to the present invention, there is provided (*S*)-α-phenyl-2-pyridineethanamine benzoate, 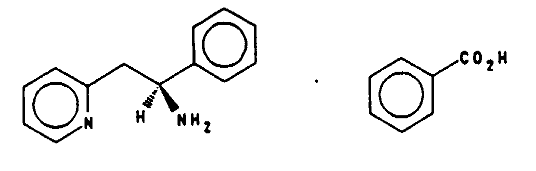 (referred to herein as "the compound of the invention") which is greater than 90% enantiopure.

By "greater than 90% enantiopure" we mean that the amine component of the salt is greater than 90% enantiopure, *i.e.* it contains less than 10% by weight of the corresponding (*R*)-enantiomer.

Preferably, the compound of the invention is more than 99% enantiopure, most preferably as close to 100% enantiopure as known methods of optical purification (*e.g.* fractional crystallization, chiral chromatography), chiral starting materials and/or chiral synthesis will allow.

(*S*)-α-phenyl-2-pyridineethanamine may be prepared by the method of Example 1 below.

The invention also provides a method of preparation of (*S*)-α-phenyl-2-pyridineethanamine benzoate which comprises precipitation from a solution of a mixture of (*S*)-α-phenyl-2-pyridineethanamine, or a salt thereof, which is greater than 90% enantiopure, and benzoic acid.

Enantiopurities may be determined by methods well known to those skilled in the art. For example, the enantiomers to be analyzed may be passed through a chiral chromatography column in normal or reverse phase mode (*e.g.* a Diacel Chiralcel OD column or a Diacel Chiralcel OD-R column respectively). Also, the enantiomers to be analyzed may be derivatized with a chiral derivatizing agent [*e.g*. *(R)-* or (*S*)-methylbenzyl isocyanate for an amine] and passed through an achiral chromatography column (*e.g.* an Alltech Adsorbosphere silica column), or the enantiomers to be analyzed may be derivatized with an achiral derivatizing agent (*e.g.* 3,5-dinitrobenzoyl chloride for an amine) and passed through a chiral chromatography column (*e.g.* a Pirkle covalent naphthylalanine column).

The compound of the invention is indicated as a pharmaceutical, in particular as an anticonvulsant and a neuroprotective agent in the treatment of neurodegenerative disorders. Specific neurodegenerative disorders that may be mentioned include stroke, cerebral ischaemia, cerebral palsy, the effects of hypoglycaemia, epilepsy, AIDS-related dementia, Alzheimer's disease, Huntington's chorea, Olivo-ponto-cerebellar atrophy, perinatal asphyxia, Parkinson's disease, anoxia, neuronal damage associated with substance abuse (for example, narcotics or cocaine), retinopathies, schizophrenia, ischaemic states after cardiac arrest or surgical operations, intoxication or injuries of the spinal cord and amyotrophic lateral sclerosis. Anticonvulsant therapy in epilepsy, and neuroprotective therapy in stroke, cerebral ischaemia and anoxia are of particular interest.

While not being limited by theory, neurodegeneration is thought to be caused or accelerated by certain excitatory amino acids found naturally in the central nervous system (CNS). Glutamate is an endogenous amino acid which has been characterized as a fast excitatory transmitter in the mammalian brain. Glutamate is also known as a powerful neurotoxin capable of killing CNS neurons under certain pathologic conditions which accompany stroke and cardiac arrest. It has been shown that the sensitivity of central neurons to hypoxia and ischaemia can be reduced by the specific antagonism of post synaptic glutamate receptors. Glutamate is characterized as a broad spectrum agonist having activity at four neuronal excitatory amino acid receptor sites. These receptor sites are named after the amino acids which selectively excite them: kainate (KA), N-methyl-D-aspartate (NMDA), quisqualate (QUIS) and 2-amino-4-phosphonobutyrate (APB). Glutamate is believed to be a mixed agonist capable of binding to and exciting all four receptor types. Thus, agents which selectively block or antagonise the action of glutamate at these receptors can prevent neurotoxic injury associated with anoxia, hypoxia or ischemia. In particular, compounds which bind to the NMDA receptor site and selectively block the action of glutamate are useful in the prevention and treatment of neurodegenerative diseases.

The pharmacological activity of the compound of the invention may be measured in the tests set out below.
a) **NMDA blocking activity** is measured by assessing a compound's ability to protect mice from convulsions induced by intravenous administration of 150mg/kg of NMDA according to the procedures of Czuczwar *et al,* (Neurotransmitters, Seizures and Epilepsy III, edited by G Nistico *et al,* Raven Press, New York 1986, pages 235-246). Groups of mice are pretreated by 30 minutes with the test compound by the intraperitoneal route and then given NMDA. Animals are observed for convulsions as defined by loss of righting reflex and appearance of tonic/clonic seizures. Animals are kept for 60 minutes after NMDA dosing and mortality is recorded.
b) **NMDA receptor antagonist activity** may be measured *in vitro* by assaying a compound's ability to inhibit binding of the receptor antagonist 10,11-dihydro-5-methyl-5H-dibenzo[a,d]-cyclohepten-5,10-imine (MK 801) to the receptor. The method is described by Foster and Wong, Br J Pharmacol 91, 403-409 (1987).
c) **NMDA and glycine receptor affinity** may also be tested in the [³H]L-glutamate and [³H]glycine binding assays following the method of Monaghan & Cotman, PNAS, 83, 7532, (1986) and Watson *et al,* Neurosci Res Comm, 2, 169, (1988).
d) **Antihypoxia activity** may be measured conveniently in mice. Groups of mice are tested at various times after the intraperitoneal administration of graded doses of the test compound. The animals' survival time in a temperature-controlled hypoxic environment (96% nitrogen and 4% oxygen) is recorded. A statistical comparison is made between coincident vehicle treated animals and the experimental group. The dose-response and minimum active dose (MAD) for compounds are obtained [A A Artu and J D Michenfelder, Anaesthesia and Analgesia, 1981, 60, 867]. Other modes of administration can also be used.
e) **Antiepileptic activity** may be measured by assessing a compound's ability to prevent the hind limb tonic extension component of the seizure in groups of mice or rats induced by maximal electroshock (MES) after oral, intraperitoneal, intravenous or subcutaneous administration, according to the procedures of the Epilepsy Branch, NINCDS as published by R J Porter, *et al,* Cleve Clin Quarterly 1984, 51, 293, and compared with the standard agents dilantin and phenobarbital.
f) **The 4-vessel occlusion (4-VO) model of stroke** is used to produce global ischaemia in the rat and is an essential technique to evaluate the effectiveness of compounds to prevent damage to areas of selective vulnerability in the brain, notably the CA1 pyramidal neurons of the hippocampus. This area is involved in the pathways for short term memory formation in both laboratory animals and humans. The procedure consists of cauterizing the vertebral arteries and isolating the carotid arteries of rats maintained under anaesthesia on day 1. On day 2 the carotids are clamped for varying periods of time, ten minutes is sufficient to destroy the CA1 neurons. The clamps are removed, reflow initiated and drugs administered at various times post reflow. Body temperature is maintained at 37°C throughout the ischaemia and recovery periods. The CA1 neurons die off over a 48-72 hour period and normally the rats are treated for at least 3 days with drug (ip, iv, or po) and at 7 days the brains are removed for histology. Rating of CA1 damage is accomplished using two methods, counting of viable CA1 neurons and scoring of degree of gross pathology [W A Pulsinelli and A Buchan, 'The NMDA receptor/ion channel: Its importance to in vivo ischemia injury to selectively vulnerable neurons', Pharmacology of Cerebral Ischemia, edited by J Krieglstein and H Oberpichler, published by Wissenschaftliche Verlagsgesellschaft, Stuttgart, 1990, p169].
g) In the **Focal Model of Stroke**, spontaneously hypertensive rats (SHR) are used as experimental subjects because of their relatively poor collateral brain circulation. A 2 hour focal ischemia is achieved in SHR by clamping the middle cerebral artery and the ipsilateral carotid while maintaining anaesthesia. Drugs can be administered (usually ip) either before or at various times after clamping the arteries or when reflow commences at 2 hours. The brains are removed 24 hours after the experiment and frozen, sectioned and drug effects toward reducing infarct volume of the cerebral cortex is determined using a custom-built computer quantification system [A M Buchan, D Xue and A Slivka, Stroke, 1992, 23, 273.]

The toxicity of the compound of the invention may be measured in the following tests.
a) **Dose ranging studies** based on those described by N W Spurling and P F Carey, 'A protocol for dose selection in repeat dose toxicity studies', poster presentation 974 at the Society of Toxicology annual meeting, Seattle, USA, 23-27 February 1992. Rats are dosed intravenously daily with progressively increasing doses of test compound until a maximum repeatable dose is found above which the incidence of convulsions and other abnormal clinical signs is unacceptable.
b) **The inverted screen test** [L L Cougenour, J R McLean, and R B Parker, Pharmacol Biochem Behav, 1977, 6, 351]. Mice are dosed with test compound and 30 minutes later are placed on a small wire platform which is inverted through an arc of 180°. Mice unable to climb to the upright position within 30 seconds are rated as failures. Using sufficient doses and numbers of animals an appropriate TD₅₀ (dose in which 50% fail) can be determined readily.
c) **The observation test for 28 behavioral signs** according to S Irwin [Psychopharmacology 1968, 13, 222]. Groups of 3 mice per dose are administered incremental amounts of test compound in the range 25-400 mg/kg and observed for 28 symptoms immediately after dosing, 30 minutes, 3 hours and 24 hours post dose.
d) **Test for Phencyclidine (PCP)-Like Behaviour**. PCP-like behaviours are a side effect of potent competitive and non-competitive NMDA receptor antagonists. In a screen to determine whether a compound possesses this liability, rats are dosed orally with test compound (expressed as multiples of the oral ED₅₀ for protection in the MES test) and placed into individual clear plastic cages and observed over a 4 hour period for any incidence of 5 characteristic behaviours associated with PCP, namely hyperactivity, ataxia, circling, head weaving and retropulsion. Five rats per treatment group are observed and compared to a control group receiving PCP. A total incidence score would be 25, *i.e.* 5 rats exhibiting all 5 behaviours. PCP at 10 times the ED₅₀ produces a score of 25 [W Koek, J H Woods, P Ornstein, 1987, Psychopharmacology, 91, 297].
e) **Gang Plank Escape Test** to measure neural impairment in rats [G E Garske *et al,* Epilepsy Research, 1991, 9, 161]. Rats are placed on a narrow board (1.25cm wide suspended 40cm above the bench top) in a well lit entry cubicle which enters a progressively darkened box connected to a dark escape cubicle at the other end (board is 63cm long). A rat is impaired if it fails to negotiate the plank. The task takes into account two known behaviours of rats, *i.e.* fear of height and seeking a dark environment.

**Linear pharmacokinetics** may be detected in rats by evaluating the area under the plasma concentration *v* time curves obtained upon single intravenous administration of test compound at increasing doses (Smith *et al,* Xenobiotica, 20, 1187-1199, 1990). Blood was removed from a jugular vein catheter at various times over a 24 hour period. The plasma was separated by centrifugation and the concentration of test compound was determined using HPLC-UV chromatography. The plasma concentration *v* time values were plotted for each dose and the area under each curve estimated. Where linear pharmacokinetics are present, the area under the plasma concentration *v* time curve for a given dose is directly proportional to the dose administered. A finding of linear pharmacokinetics in rats indicates that linear pharmacokinetics would be found in humans (Leander *et al,* Epilepsia, 33, 696-704, 1992, at p703).

According to another aspect of the invention there is provided a method of treatment of a neurodegenerative disorder, which comprises administering a therapeutically effective amount of the compound of the invention to a patient. Of particular interest is such a method in which the dose of the compound administered is linearly proportional to the blood plasma concentration of the compound desired.

For the above-mentioned uses the dosage administered will, of course, vary with the compound employed, the mode of administration and the treatment desired. However, in general, satisfactory results are obtained when the compound of the invention are administered at a daily dosage of from about 0.lmg to about 20mg per kg of animal body weight, preferably given in divided doses 1 to 4 times a day or in sustained release form. For man, the total daily dose is in the range of from 5mg to 1,400mg, more preferably from 10mg to 100mg, and unit dosage forms suitable for oral administration comprise from 2mg to 1,400mg of the compound admixed with a solid or liquid pharmaceutical carrier or diluent.

The compound of the invention may be used on its own or in the form of appropriate medicinal preparations for enteral or parenteral administration. According to a further aspect of the invention, there is provided a pharmaceutical formulation including preferably less than 80% and more preferably less than 50% by weight of the compound of the invention in admixture with a pharmaceutically acceptable adjuvant, diluent or carrier.

Examples of diluents and carriers are:
for tablets and dragees: lactose, starch, talc, stearic acid;
for capsules: tartaric acid or lactose;
for injectable solutions: water, alcohols, glycerin, vegetable oils;
for suppositories: natural or hardened oils or waxes.

An adjuvant of particular interest when the compound of the invention is to be used in the treatment of Parkinson's disease is L-dopa.

According to a further aspect of the invention, there is provided the use of the compound of the invention as active ingredient in the manufacture of a medicament for the treatment of a neurodegenerative disorder.

The compound of the invention may also have the advantage that it is less toxic, more efficacious, is longer acting, has a broader range of activity, is more potent, produces fewer side effects, is more easily absorbed or has other more useful pharmacological properties, than compounds previously indicated in the therapeutic fields mentioned above.

The invention is illustrated by the following examples.

### Example 1

### Preparation of (S)-α-phenyl-2-pyridineethanamine dihydrochloride

### a) α-Phenyl-2-pyridineethanamine dihydrochloride

To a cooled (0°C) solution of benzaldehyde (34.24g, 0.323 moles) in 600ml of tetrahydrofuran (THF) was added lithium bis(trimethylsilyl)-amide (LHMDS) (323ml of a 1.0M solution in THF, 0.323 moles) dropwise over 30 minutes. This mixture was stirred at 0°C for three hours.

In a separate round bottom flask containing a cooled (-78°C) solution of 2-picoline (30.0g, 0.323 moles) in THF (600ml) was added n-butyllithium (n-BuLi) (129.2ml of a 2.5M solution in hexane) over twenty minutes.

The first reaction mixture was allowed to warm to 0°C and remain there for an additional forty minutes. The second reaction mixture (containing the lithiated anion of 2-picoline) was cannulated into the first reaction mixture over 20 minutes. After 30 additional minutes the cold bath was removed and the mixture was allowed to warm to ambient temperature. After an additional one hour, the reaction mixture was poured into a separating funnel charged with ice (11) and 12 N HCI (200ml). The aqueous layer was washed with 3x200ml of diethyl ether (Et₂O) and then basified with 25% NaOH solution in water. The aqueous layer was extracted with 2x200ml of chloroform, the chloroform extracts dried over MgSO₄, filtered and concentrated *in vacuo.* The residue was dissolved in ethyl acetate (EtOAc) and acidified with a saturated solution of HCl/EtOAc. The solution was diluted with Et₂O and the resulting white solid filtered and dried *in vacuo* to give the subtitle compound (37.08g, 43%), mp = 206-208°C.

### b) (S)-α-Phenyl-2-pyridineethanamine dihydrochloride

To a solution of racemic α-phenyl-2-pyridineethanamine (the free base of the product of step (a), obtained by neutralizing an aqueous solution of the product of step (a) with a 25% NaOH solution in water and extracting with chloroform) (10.96g, 0.0553 moles) in EtOAc (400ml) was added a solution of S(+)-mandelic acid (8.41g, 0.0553 moles) in EtOAc (300ml). The resulting precipitate was recrystallized from hot EtOAc (500ml) an additional three times. The salt was basified with a 25% NaOH solution in water, extracted with 3x100ml of chloroform, dried over MgSO₄, filtered and concentrated *in vacuo*. The residue was dissolved in EtOAc (300ml) and acidified with a saturated solution of HCl/EtOAc. The resulting white solid was filtered and dried *in vacuo* to give (-)-α-phenyl-2-pyridineethanamine dihydrochloride (5.5g), mp = 220-222°C, [α]_{D} = -87.3° (c = 1.0, CH₃OH).

The filtrate from the initial precipitation was neutralized with 25% NaOH solution in water, extracted with 2x250ml of CHCl₃, dried over MgSO₄, filtered and concentrated *in vacuo.* The residue was dissolved in EtOAc (500ml) and to this solution was added a solution of R(-)-mandelic acid (6.5g, 0.043 moles) in EtOAc (500ml). The precipitate was filtered off and recrystallized an additional three times. The salt was basified with 25% NaOH solution in water, extracted with 3x100ml of chloroform, dried over MgSO₄, filtered and concentrated *in vacuo.* The residue was dissolved in EtOAc (300ml) and acidified with a saturated solution of HCl/EtOAc. The resulting white solid was filtered and dried *in vacuo* to give the title compound (3.84g), mp = 220-222°C, [α]_{D} = +87.1° (c = 1.1, CH₃OH).

The enantiopurity may be determined by derivatizing either the mandelic acid or dihydrochloride salt with enantiopure (greater than 99.5%) methylbenzyl isocyanate, and then analyzing by HPLC using a normal phase column with ethanol/hexane [6:94] as solvent. The enantiopurity of the enantiomers obtained above was shown to be greater than 99.5%.

X-ray crystallography showed the (+)-enantiomer to have (*S*)-absolute stereochemistry.

### Example 2

### Preparation of (S)-α-phenyl-2-pyridineethanamine benzoate from (S)-α-phenyl-2-pyridineethanamine dihydrochloride

To a solution of the title compound of Example 1 (7.8g) in ethyl acetate (20ml) was added benzoic acid (4.7g) in hot ethyl acetate (40ml). The mixture was allowed to cool to ambient temperature and stirred for one hour. The resulting white solid was filtered off, washed with ethyl acetate and dried *in vacuo* to yield the title compound (10.4g). mp = 134-136°C; [α]_{D} = +47.78° (c=1.02965, CH₃OH, 23°C); enantiopurity of the amine moiety 99.8% (as determined by chiral HPLC).

### Example 3

The stability of the title compound of Example 1 and the title compound of Example 2 to moisture was investigated. The former compound was found to deliquesce at a relative humidity of 80%, whereas the latter compound had not started to deliquesce at a relative humidity of 97%.

### Example 4

The title compound of Example 2 was found to inhibit hind limb tonic extension in mice by 50% (ED₅₀) induced by maximal electroshock (MES, test described above) when administered orally at a dose of less than 10mg/kg.

## Claims

1. (*S*)-α-phenyl-2-pyridineethanamine benzoate.

2. (*S*)-α-phenyl-2-pyridineethanamine benzoate as claimed in claim 1 which is greater than 90% enantiopure.

3. (*S*)-α-phenyl-2-pyridineethanamine benzoate as claimed in claim 2 which is greater than 99% enantiopure.

4. (*S*)-α-phenyl-2-pyridineethanamine benzoate as claimed in claim 3 which is essentially 100% enantiopure.

5. A pharmaceutical formulation including (*S*)-α-phenyl-2-pyridineethanamine benzoate as defined in any one of claims 1 to 4, in admixture with a pharmaceutically acceptable adjuvant, diluent or carrier.

6. (*S*)-α-phenyl-2-pyridineethanamine benzoate, as defined in any one of claims 1 to 4, for use as a pharmaceutical.

7. The use of (*S*)-α-phenyl-2-pyridineethanamine benzoate, as defined in any one of claims 1 to 4, as active ingredient in the manufacture of a medicament for the treatment of a neurodegenerative disorder.

8. A process for the production of (*S*)-α-phenyl-2-pyridineethanamine benzoate as defined in any one of claims 1 to 4, which comprises precipitation from a solution of a mixture of (*S*)-α-phenyl-2-pyridineethanamine, or a salt thereof, which is greater than 90% enantiopure, and benzoic acid.

## Patentansprüche

1. (S)-α-Phenyl-2-pyridinethanamin-benzoat.

2. (S)-α-Phenyl-2-pyridinethanamin-benzoat nach Anspruch 1, das über 90% enantiomerenrein ist.

3. (S)-α-Phenyl-2-pyridinethanamin-benzoat nach Anspruch 2, das über 99% enantiomerenrein ist.

4. (S)-α-Phenyl-2-pyridinethanamin-benzoat nach Anspruch 3, das im wesentlichen 100% enantiomerenrein ist.

5. Pharmazeutische Formulierung, enthaltend (S)-α-Phenyl-2-pyridinethanamin-benzoat nach einem der Ansprüche 1 bis 4 zusammen mit einem pharmazeutisch unbedenklichen Hilfsstoff, Verdünnungsmittel oder Träger.

6. Verwendung von (S)-α-Phenyl-2-pyridinethanaminbenzoat nach einem der Ansprüche 1 bis 4 als Pharmazeutikum.

7. Verwendung von (S)-α-Phenyl-2-pyridinethanaminbenzoat nach einem der Ansprüche 1 bis 4 als Wirkstoff bei der Herstellung eines Arzneimittels zur Behandlung einer neurodegenerativen Erkrankung.

8. Verfahren zur Herstellung von (S)-α-Phenyl-2-pyridinethanamin-benzoat nach einem der Ansprüche 1 bis 4, bei dem man aus einer Lösung eines Gemischs aus (S)-α-Phenyl-2-pyridinethanamin oder einem seiner Salze mit einer Enantiomerenreinheit über 90% und Benzoesäure fällt.

## Revendications

1. Benzoate de (*S*)-α-phényl-2-pyridineéthanamine.

2. Benzoate de (*S*)-α-phényl-2-pyridineéthanamine suivant la revendication 1, qui est l'énantiomère d'une pureté supérieure à 90 %.

3. Benzoate de (*S*)-α-phényl-2-pyridineéthanamine suivant la revendication 2, qui est l'énantiomère d'une pureté supérieure à 99%.

4. Benzoate de (*S*)-α-phényl-2-pyridineéthanamine suivant la revendication 3, qui est l'énantiomère d'une pureté de sensiblement 100%.

5. Composition pharmaceutique comprenant du benzoate de (*S*)-α-phényl-2-pyridineéthanamine, tel que défini dans l'une quelconque des revendications 1 à 4, en mélange à un véhicule ou excipient, diluant ou adjuvant pharmaceutiquement acceptable.

6. Benzoate de (*S*)-α-phényl-2-pyridineéthanamine, tel que défini dans l'une quelconque des revendications 1 à 4, en vue de l'utilisation, à titre de composé pharmaceutique.

7. Utilisation du benzoate de (*S*)-α-phényl-2-pyridineéthanamine, tel que défini dans l'une quelconque des revendications 1 à 4, à titre d'ingrédient actif pour la fabrication d'un médicament destiné au traitement d'un trouble neuro-dégénératif.

8. Procédé de production du benzoate de *(S)*-α-phényl-2-pyridineéthanamine, tel que défini dans l'une quelconque des revendications 1 à 4, qui comprend la précipitation à partir d'une solution d'un mélange de (*S*)-α-phényl-2-pyridineéthanamine, ou d'un sel de celle-ci, qui est l'énantiomère d'une pureté supérieure à 90% et d'acide benzoïque.
